(19) Europäisches Patentamt
European Patent Office
Office européen des brevets

(11) EP 2 474 569 A1

(12) **EUROPEAN PATENT APPLICATION**
published in accordance with Art. 153(4) EPC

(43) Date of publication:
**11.07.2012 Bulletin 2012/28**

(21) Application number: **10813655.7**

(22) Date of filing: **26.08.2010**

(51) Int Cl.:
***C08J 9/28*** *(2006.01)* ***A61K 31/715*** *(2006.01)*
***A61P 41/00*** *(2006.01)*

(86) International application number:
**PCT/JP2010/064451**

(87) International publication number:
**WO 2011/027706 (10.03.2011 Gazette 2011/10)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **02.09.2009 JP 2009203031**

(71) Applicant: **Terumo Kabushiki Kaisha**
**Tokyo 151-0072 (JP)**

(72) Inventors:
- **CHINO, Naotaka**
  **Ashigarakami-gun**
  **Kanagawa 259-0151 (JP)**
- **KAI, Miho**
  **Ashigarakami-gun**
  **Kanagawa 259-0151 (JP)**

(54) **POROUS STRUCTURE**

(57) The objective of this description is to provide a means for improving the solubility of a polysaccharide in water. Disclosed is a porous structure including a polysaccharide and having a multitude of pores wherein an average pore size of the pores is not smaller than 40 μm and a ratio of the number of pores having a pore size not smaller than 50 μm to the total number of the pores is not less than 30%.

Fig. 1

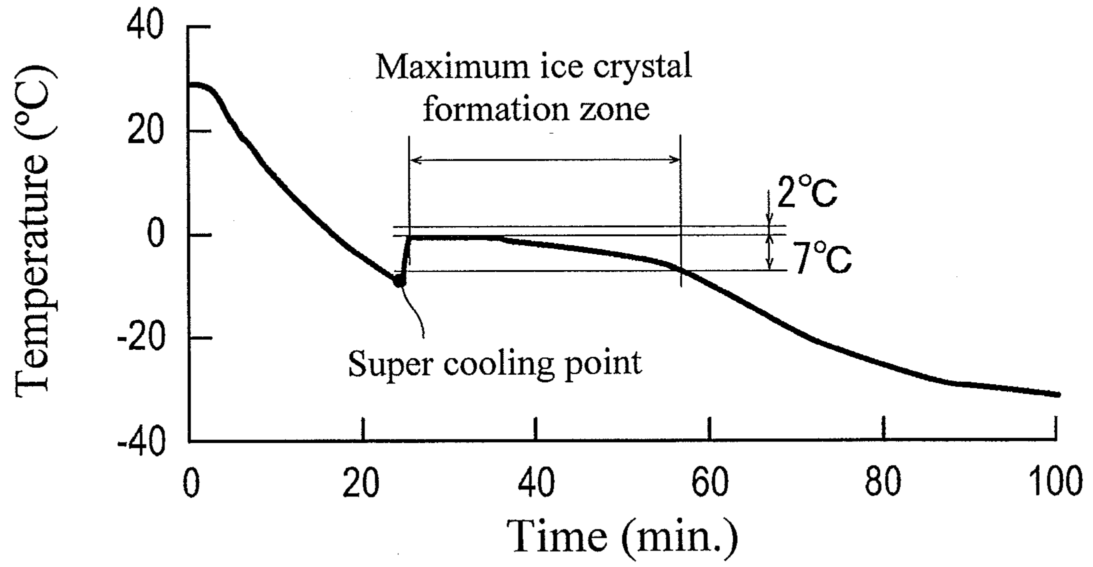




Printed by Jouve, 75001 PARIS (FR)

## Description

### Technical Field

This invention relates to a porous structure. More particularly, the invention relates to an improvement of improving solubility of polysaccharides in water.

### Background Art

In surgical procedures, there may be some case where living tissues are damaged by surgery operation. It is known that when living tissues are exposed to air by incision, the living tissues are dried or oxidized, resulting in tissue damage. When the damaged tissues undergo inflammation after the operation, there is the possibility of adhesion of tissues that would be normally separated from each other. Such adhesion of tissues after the operation may cause severe complications such as of intestinal obstruction, infertility or the like, for example, in the abdominal region. Hence, there have been developed a variety of adhesion-preventing materials that cover the damaged portion of living tissues so as to prevent adhesion.

Currently known adhesion-preventing materials are made mainly of bio-derived polymer materials such as polysaccharides, polypeptides and the like, which are unlikely to give adverse influences on living body, with a variety of their forms including a powder, a sheet, a jelly, a liquid and the like. Above all, attention has been paid especially to liquid adhesion-preventing materials from the standpoint of user-friendliness in that a desired region of living tissues can be covered by spraying.

Among such adhesion-preventing materials, an adhesion-preventing material disclosed, for example, in Patent Document 1 is made of a crosslinking polysaccharide derivative wherein an active ester group capable of reaction with an active hydrogen-containing group is introduced into the side chain of a polysaccharide. This crosslinking polysaccharide derivative is able to form a crosslinked product through a covalent bond between the active ester group and the active hydrogen-containing group by contact with water under alkaline conditions.

Further, the Patent Document 1 also discloses an adhesion-preventing method as a preferred embodiment wherein an aqueous solution containing the above-mentioned crosslinking polysaccharide derivative is applied onto a desired region of living tissue and an aqueous solution containing a pH adjuster for making alkaline conditions is sprayed thereover to cause the crosslinking polysaccharide derivative to be gelled.

Furthermore, an applicator has been developed (for example, in Patent Document 2) wherein two-component medicinal solutions such as of the above-mentioned crosslinking polysaccharide derivative-containing aqueous solution and a pH adjuster-containing solution can be sprayed simultaneously. Using such an applicator, an adhesion-preventing material can be applied onto a desired region by one time spraying. It will be noted that a polysaccharide dissolved in water is more liable to become deteriorated than a dry solid polysaccharide, for which it is favorable to prepare a polysaccharide aqueous solution on a case-by-case basis by adding water to a dry solid polysaccharide at an operation site.

### Prior Art Documents

### Patent Documents

Patent Document 1: Pamphlet of International Laid-open No. 2005/087289 (U.S. Patent Application Publication No. 2008-058469 Specification)

Patent Document 2: Japanese Patent Laid-open No. 2008-289986 (U.S. Patent Application Publication No. 2008-294099 Specification)

### Summary of the Invention

### Problem to Be Solved by the Invention

However, polysaccharides that are favorably usable as such an adhesion-preventing material as set out above contain a hydrophobic group or have a great molecular weight, so that they are generally sparingly soluble in water. Accordingly, it takes much time before a dry solid polysaccharide is dissolved in water, making it difficult to rapidly prepare a polysaccharide aqueous solution. Thus, adhesion-preventing materials containing a water sparingly-soluble polysaccharide have had a problem in that they are not responsible for quick application.

Therefore, the invention has for its object the provision of a means for improving solubility of polysaccharides in water.

Means for Solving the Problem

We have made intensive studies so as to solve the above problem. As a result, it has been found that when conditions of freeze-drying a polysaccharide are appropriately controlled, there can be obtained a porous structure that has a given pore size and is significantly improved in solubility in water, thereby completing the invention. More particularly, the above object of the invention can be achieved by the following (1) to (5).

(1) A porous structure including a polysaccharide and having a multitude of pores wherein an average pore size of the pores is not smaller than 40 μm and a ratio of the number of pores having a pore size not smaller than 50 μm to the total number of the pores is not less than 30%.

(2) A porous structure obtained by freeze-drying a polysaccharide aqueous solution containing a polysaccharide according to the following steps (A) and (B): the freezing step (A) of cooling the polysaccharide aqueous solution down to over a supercooling point and subsequently obtaining ice crystals of the polysaccharide aqueous solution over a passage time through a maximum ice crystal formation zone of not less than five minutes; and the drying step (B) of removing water contained in the ice crystals by sublimation under reduced pressure.

(3) The porous structure as defined in (1) or (2), wherein the polysaccharide has at least one selected from the group consisting of a carboxyl group, an active ester group, a carboxylate salt, an amino group and an aldehyde group.

The porous structure as defined in any of (1) to (3), wherein a standard deviation σ of the pores is at 5 to 30 μm.

A method for preparing a porous structure by freeze-drying a polysaccharide aqueous solution according to the following steps (A) and (B): the freezing step (A) of cooling the polysaccharide aqueous solution down to over a supercooling point and subsequently obtaining ice crystals of the polysaccharide aqueous solution over a passage time through a maximum ice crystal formation zone of not less than five minutes; and the drying step (B) of removing water contained in the ice crystals by sublimation under reduced pressure.

Effect of the Invention

According to the invention, solubility of polysaccharides in water can be improved, thus enabling a polysaccharide aqueous solution to be rapidly prepared at an operation site.

Brief Description of the Drawings

[Fig. 1]
Fig. 1 is a graph showing a typical cooling curve of a polysaccharide aqueous solution in a method for preparing a porous structure of an embodiment of the invention.
[Fig. 2]
Fig. 2 is a graph showing a cooling graph in case where polysaccharide aqueous solutions in Examples 1 and 2 are frozen.
[Fig. 3]
Fig. 3 is a graph showing a cooling curve in case where a polysaccharide aqueous solution in Comparative Example 1 is frozen.
[Fig. 4]
Fig. 4 is a graph showing a cooling curve in case where a polysaccharide aqueous solution in Comparative Example 2 is frozen.
[Fig. 5]
Fig. 5 is a scanning electron micrograph (SEM) image of a cut surface of a porous structure obtained in Example 1.
[Fig. 6]
Fig. 6 is a scanning electron micrograph (SEM) image of a cut surface of a porous structure obtained in Example 2.
[Fig. 7]

Fig. 7 is a scanning electron micrograph (SEM) image of a cut surface of a porous structure obtained in Comparative Example 1.
[Fig. 8]
Fig. 8 is a scanning electron micrograph (SEM) image of a cut surface of a porous structure obtained in Comparative Example 2.

Mode for Carrying Out the Invention

A preferred embodiment of the invention is now described. This embodiment relates to a porous structure including a polysaccharide and having a multitude of pores. The average size of the pores should be not smaller than 40 μm and a ratio of the number of pores whose pore size is not smaller than 50 μm to the total number of the pores is not less than 30%.

<Porous structure>

The porous structure of this embodiment essentially contains a polysaccharide. The term "polysaccharide" used herein means a polymer wherein monosaccharide molecules are bonded through glycoside bond and which has a molecular weight of not less than 1000.

The monosaccharide constituting a polysaccharide is not critical in type and includes, for example, ribose, xylose, arabinose, glucose, mannose, galactose, fructose, sorbose, rhamnose, fucose and ribodeose, and monosaccharide derivatives wherein an arbitrary functional group is introduced into these monosaccharides. Polysaccharides may be formed of only one type of monosaccharide selected among them, or may be formed of a combination of two or more, and may be linear or may contain a branch. Naturally occurring polysaccharides may be used, or synthetic ones may be used. Of these polysaccharides, polysaccharides containing glucose or a glucose derivative are preferred in view of the results of administration to living body. More specifically, it is more preferred to contain at least one polysaccharide selected from the group consisting of dextrin, dextran and cellulose, and derivatives thereof.

The polysaccharide related to the invention should preferably have at least one selected from the group consisting of a carboxyl group, an active ester group, a carboxylate salt, an amino group and an aldehyde group. Such a functional group is able to establish a crosslinking point, at which an ester bond or an amide bond is formed, and is thus suitable as a constituent component of an adhesion-preventing material. If these functional groups are contained as an adhesion-preventing material, the functional groups and hydroxyl group contained in a polysaccharide form an ester bond or amide bond along with a hydroxyl group present in the surface of a living tissue and are thus crosslinked when an aqueous solution containing the polysaccharide is kept under alkaline conditions. In doing so, the adhesion-preventing material becomes gelled, thereby enabling the surface of the living tissue to be covered therewith.

One instance of polysaccharide having a carboxyl group is of the form wherein a carboxyalkyl group is introduced into a hydroxyl group of polysaccharide. Such carboxyalkyl groups are preferably carboxyalkyl groups having 2 to 5 carbon atoms and specifically include a carboxymethyl group, a carboxyethyl group, a carboxypropyl group, a carboxyisopropyl group and a carboxybutyl group. Of these a carboxymethyl group or carboxyethyl group is preferred, of which a carboxymethyl group is more preferred.

An example of polysaccharide having an active ester group is of the form wherein a carboxyl group contained in a carboxyl group-bearing polysaccharide and an N-hydroxyamine compound are esterified. Examples of the N-hydroxyamine compound include N-hydroxysuccinimide, N-hydroxynorbornene-2,3-dicarboxylic imide, 2-hydroxyimino-2-cyanoacetic ethyl ester, 2-hydroxyimino-2-cyanoacetic amide, N-hydroxypiperidine and the like, of which N-hydroxysuccinimide is preferred.

Polysaccharides having a carboxylate salt are ones wherein a carboxylic acid ion left after removal of a hydrogen ion from a carboxyl group present in a carboxyl group-bearing polysaccharide is ionically bonded with a cation other hydrogen ion. The cation includes an alkali metal ion, an alkaline earth metal, and a quaternary ammonium ion such as a tetra(n-butyl)ammonium or tetra(n-propylmethyl)ammonium ion.

Polysaccharides having an amino group include, for example, ones containing glucosamine and an amino sugar such as glucosamine, and isourea intermediate-containing polysaccharides (polysaccharides containing a structure of the following chemical formula 1)

[Chemical Formula 1]

[Chemical Formula 1]

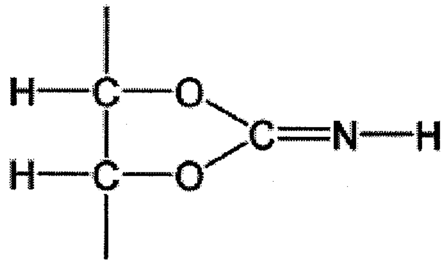

Polysaccharides having an aldehyde group include, for example polysaccharides containing an aldose at the reducing end of polysaccharide.

Polysaccharides having such functional groups as set out above may be naturally occurring ones, or may be prepared according to chemical or biological techniques. For the preparation of polysaccharides having these functional groups, hitherto known means may be appropriately adopted.

It will be noted that as a matter of course, the porous structure of this embodiment may contain constituent ingredients other than polysaccharide. The constituent ingredients other than polysaccharide includes oligosaccharides having less than 1000 molecular weight although not limited thereto.

The weight average molecular weight of polysaccharide is not critical and is preferably at 50000 to 200000, more preferably at 50000 to 100000. By using a polysaccharide whose molecular weight is within such a range as indicated above, where the porous structure of this embodiment is employed as a constituent ingredient of the adhesion-preventing material, a crosslinked polysaccharide can have a good gel hardness. It will be noted that in the present specification, the weight average molecular weight adopted is one obtained by GPC.

The porous structure of this embodiment has a multitude of pores. The average pore size is not smaller than 40 μm, preferably 40 μm to 200 μm. As will be described hereinafter, the porous structure of this embodiment is prepared by obtaining ice crystals of a polysaccharide aqueous solution over a passage time through a maximum ice crystal formation zone of not less than five minutes and subjecting the ice crystals to sublimation under reduced pressure. According to such a preparation method, ice crystals grow uniformly, so that it is substantially difficult that the average pore size exceeds 200 μm. The term "pore" used herein means a void portion surrounded by walls made of the constituent ingredient of the polysaccharide-containing porous structure. The term "multitude of pores" means the existence of not smaller than 500 pores per square centimeter. The term "pore size" means a length of the void. The term "average pore size" means an average value of the pore sizes. It will be noted that the values of "pore size" and "average pore size" adopted in this specification are those values obtained by measuring methods used in examples appearing hereinafter, respectively. The porous structure of this embodiment should essentially have a ratio of the number of pores having a pore size of not smaller than 50 μm to the total number of pores of not less than 30%, preferably 40 to 80%. In this way, the porous structure of the embodiment has a great number of pores that are larger in size than prior art counterparts and thus, when a polysaccharide is dissolved by contact with water, gelation in the pores is suppressed thereby enabling dissolution within a short time.

The standard deviation σ of pore sizes of the porous structure is preferably at 5 to 30 μm, more preferably at 5 to 20 μm. If the standard deviation σ of the pore size is within this range, very fine pores are small in number and solubility in water becomes good.

<Method of preparing a porous structure>

The porous structure of the embodiment can be readily prepared by freeze-drying of a polysaccharide aqueous solution containing the above-indicated polysaccharide, including the two steps of freezing step (A) and drying step (B). A preferred preparation method of a porous structure of the embodiment is now described.

Initially, a polysaccharide aqueous solution containing a polysaccharide is prepared. Water used upon preparation of the polysaccharide aqueous solution is not critical. Preferably, there are used RO water, distilled water and water for injection, which contain a reduced amount of impurities. The concentration of the polysaccharide aqueous solution is not critical. In general, the polysaccharide aqueous solution is prepared by using 6 to 20 g, preferably 8 to 15 g, of water per unit gram of polysaccharide. The manner of dissolution wherein a polysaccharide is dissolved in water is not critical. Generally, dissolution is carried out under agitation. In this connection, however, in so far as no adverse

influence is significantly given on the chemical characteristics of polysaccharide, dissolution may be carried out by application of heat.

Next, in the freezing step (A), the thus prepared polysaccharide aqueous solution is cooled to form ice crystals of the polysaccharide aqueous solution. In the course of converting the polysaccharide aqueous solution to ice crystals, it is essential that after the polysaccharide aqueous solution has passed through a supercooling point, the ice crystals result over a time of passage through the maximum crystal formation zone of not less than five minutes. The passage time through the maximum crystal formation zone is preferably not less than ten minutes, more preferably not less than 15 minutes. It should be noted that the upper limit of the passage time through maximum ice formation zone is influenced by the amount of the polysaccharide aqueous solution or the cooling capacity of a cooling system and is not thus critical. In view of the freezing step being completed within a shorter time, the upper limit is preferably less than 180 minutes, more preferably less than 60 minutes. In this specification, the term "supercooling point" means a point at which a liquid in a supercooled condition is solidified in the course of cooling the liquid and as shown in Fig. 1, the inclination of a cooling curve changes from minus to plus. The term "passage time through a maximum ice crystal formation zone" used herein means such a time that in a cooling curve as shown in Fig. 1, the curve exists during the time of from a temperature at the time when the inclination of the curve changes to plus after passage through the supercooling point and subsequently changes to minus again to a temperature region of -7 to +2°C. In this temperature region, the solution is solidified to form ice crystals. It is essential that the passage time through the maximum ice crystal formation zone be not less than five minutes, preferably not less than ten minutes. By the formation of ice crystals of a polysaccharide aqueous solution through such a cooling step, the pore size of the porous structure can be appropriately controlled.

Although the cooling conditions of the polysaccharide aqueous solution in the freezing step may depend, more or less, on the amount of the polysaccharide aqueous solution and the characteristics of cooling system, the freezing step, which has a supercooling point and a passage time through the maximum crystal formation zone of not less than five minutes, can be attained by gradually lowing a cooling temperature from 25 to -30°C over a time of about 60 minutes.

The cooling system used for the cooling is not critical in type and should preferably be a cooling system capable of controlling a cooling temperature so as to achieve the freezing step as set out above. Such a system as to control the cooling temperature and cooling time by program is more preferred. In order to smoothly carry out the freezing step and a subsequent drying step, the use of a freezing-drying apparatus is much more preferred.

After having obtained the ice crystals of the polysaccharide aqueous solution in the freezing step (A), water present in the ice crystals is removed by sublimation under a reduced pressure in the drying step (B). The reduced pressure conditions are not critical and are generally at 3 to 20 Pa. As a matter of course, it is necessary to keep the ice crystals at about -30 to -20°C so as to permit the water in the ice crystals to be sublimated without liquefaction. It will be noted that a drying apparatus may be one that is capable of reduced-pressure drying under cooling, and known apparatus can be appropriately adopted therefor.

## Examples

The features and effects of the invention are illustrated by way of the following examples and comparative examples. In this regard, however, the technical range of the invention should not be construed as limited only to the following examples.

<Preparation of porous structure>

[Example 1]

(Preparation of polysaccharide aqueous solution)

0.75 g of trehalose serving as a stabilizer was weighed in a 16 ml vial, to which 10 g of RO water (i.e. water from which impurities were removed through a reverse osmosis film) for dissolution. 1.5 g of carboxymethyl dextrin (weight average molecular weight: 70000) was added to the solution, followed by agitation at room temperature (25°C) at 1400 rpm for ten minutes by use of a vortex mixer.

(Freeze-drying)

The above vial was set in an aluminum block (16 holes) and a rubber stopper was attached thereto in a half-open state. This was disposed in a freeze drying apparatus (DER-5N-A, made by Ulvac Inc.) whose shelf temperature had been preliminarily set at -35°C. A thermocouple was inserted into the vial, so that the temperature of the polysaccharide aqueous solution could be monitored.

The polysaccharide aqueous solution was frozen by setting the shelf temperature at -35°C and cooling for ten

hours [freezing step]. It will be noted that a final freezing temperature ten hours after commencement of the freezing was -30°C.

Subsequently, water in the vial was dried up by sublimation according to the program of the following Table 1 to provide a porous structure [drying step].

[Table 1]

[Table 1]

| | Shelf Temperature [°C] | Time [Minutes] | CT temperature [°C] | Degree of vacuum [Pa] |
|---|---|---|---|---|
| Step 1 | -30 | 600 | <-40 | <10 |
| Step 2 | -25 | 600 | | |
| Step 3 | -20 | 600 | | |
| Step 4 | -15 | 600 | | |
| Step 5 | -10 | 300 | | |
| Step 6 | -5 | 300 | | |
| Step 7 | 0 | 60 | | |
| Step 8 | 5 | 300 | | |
| Step 9 | 20 | 300 | | |

[Example 2]

A porous structure was prepared in the same manner as in Example 1 except that RO water used in the above example (preparation of polysaccharide aqueous solution) was changed to 14 g. It will be noted that a final freezing temperature 10 hours after commencement of freezing in the freezing step was -30°C.

[Comparative Example 1]

(Preparation of polysaccharide aqueous solution)

0.25 g of trehalose was weighed in a 16 ml vial, to which 2.7 g of RO water was added for dissolution. 0.5 g of carboxymethyl dextrin (weight average molecular weight: 70000) was added to the solution, followed by agitation at room temperature (25°C) at 1400 rpm for ten minutes by use of a vortex mixer.

(Freeze-drying)

The above vial was set in an aluminum block (16 holes) that had been cooled to -45°C beforehand, and a rubber stopper was attached thereto in a half-open state. This was disposed in a freeze drying apparatus (DER-5N-A, made by Ulvac Inc.) whose shelf temperature had been preliminarily set at -35°C. A thermocouple was inserted into the polysaccharide aqueous solution in the vial, so that the temperature of the polysaccharide aqueous solution being frozen could be monitored.

The polysaccharide aqueous solution in the vial was frozen by setting the shelf temperature at -35°C and cooling for ten hours [freezing step]. It will be noted that a final freezing temperature ten hours after commencement of the freezing was -30°C.

Subsequently, water in the vial was dried up by sublimation according to the program of the above-indicated Table 1 [drying step].

[Comparative Example 2]

A porous structure was prepared in the same manner as in Comparative Example 1 except that in the above [Comparative Example 1] (preparation of polysaccharide aqueous solution), trehalose was changed to 0.7 g, RO water changed to 10 g and carboxymethyl dextrin changed to 1.5 g, respectively.

<Measurement of temperature upon freezing of polysaccharide aqueous solution>

In the above examples and comparative examples, the freezing temperatures of the polysaccharide aqueous solutions were monitored by use of the thermocouple. The temperature with time was graphically plotted. The graphs of Examples 1 and 2 are shown in Fig. 2, the graph of Comparative Example 1 shown in Fig. 3, and the graph of Comparative Example 2 shown in Fig. 4, respectively. From the graphs, the presence or absence of supercooling point and the passing time through a maximum ice crystal formation zone were confirmed. The results are shown in Table 2 below.

<Calculation of average pore size>

The porous structures obtained in the above examples and comparative examples were, respectively, cut out into a size of 1 cm square and the surface of the cut face was observed at 100 magnifications though a scanning electron microscope (SEM). The thus observed SEM images are shown in Figs. 5 to 8.

The SEM images were each printed on a paper sheet. Two portions corresponding to a 0.5 mm square cut surface of the porous structure were selected. The pore sizes of all pores existing in the portions were measured. When the pore was circular, its diameter was provided as a pore size and with respect to other shapes, a pore was surrounded with a parallelogram sufficient to make a minimum area and a value obtained by dividing the long side plus the short side of the parallelogram by two was determined as a pore size. The average value of all the pore sizes obtained in this way was provided as an average pore size. The results are shown in Table 2.

<Ratio of pores having a pore size of not smaller than 50 μm>

A ratio of the number of pores having a pore size of not smaller than 50 μm to the total number of pores existing in the two selected portions indicated above was calculated. The results are shown in Table 2 below.

<Calculation of standard deviation σ of pore sizes>

The standard deviation σ was calculated from the pore sizes and average pore size of all the pores determined above. The results are shown in Table 2 below.

<Measurement of density>

The bottom area and height of cylindrical porous structures obtained in the above examples and comparative examples were measured and the volume of the porous structure was calculated. In addition, the weight of the porous structure was also measured. The thus measured weight was divided by the volume to calculate a density of the structure, with the results shown in Table 2 below.

<Measurement of porosity>

0.5 $cm^3$ of ethanol was dropped over the respective porous structures used in the above <measurement of density>, from which a volume of ethanol absorbed in the structure was determined. The thus determined volume was substituted into the following formula 1 to calculate a porosity of the porous structure. The results are shown in Table 2 below.

[Mathematical Formula 1]

[Formula 1]

$$\text{Porosity } [\%] = \frac{\text{volume of ethanol } [cm^3]}{\text{volume of porous structure } [cm^3]} \times 100$$

<Solubility test>

0.15 g of the respective porous structures obtained in the above examples and comparative examples was cut out and placed in a polypropylene test tube. At room temperature (25°C), 0.35 g of RO water was added to the test tube

and immediately agitated by means of the Vortec to measure a time before dissolution. The presence or absence of lumps being formed during the dissolution was confirmed. The results are shown in Table 2 below.

[Table 2]

| | Freezing conditions | | Physics of porous structure | | | | | Solubility | |
|---|---|---|---|---|---|---|---|---|---|
| | Passing time through maximum ice crystal formation zone [minutes] | Super-cooling point | Density [g/cm$^3$] | Porosity [%] | Average pore size [μm] | Ratio of pores having a pore size of not smaller than 50 μm [%] | Standard deviation σ [μm] | Dissolution time [seconds] | Formation of lumps |
| Example 1 | 15 | Yes | 0.23 | 76 | 43 | 43 | 15 | 10 | No |
| Example 2 | 15 | Yes | 0.15 | 79 | 45 | 38 | 18 | 10 | No |
| Comparative Example 1 | ... .*1 | No | 0.27 | 76 | 19 | 0 | 10 | 45 | Yes |
| Comparative Example 2 | ... . *1 | Yes | 0.23 | 76 | 21 | 0 | 9 | 35 | Yes |

*1 Meaning that no passing time through maximum ice crystal formation zone was observed.

From the results of Table 2, it will be seen that the porous structures of Examples 1 and 2, which were subjected to the freezing step carried out as having a supercooling point and a passing time through the maximum ice crystal formation zone of not less than five minutes, have an average pore size of not smaller than 40 μm and a ratio of pores having a pore size of not smaller than 50 μm being not less than 35%. The porous structures of these examples had a dissolution time that was as very short as 10 seconds, and no formation of lumps during the dissolution was found.

On the other hand, in Comparative Examples 1 and 2, although the vial was set in the aluminum block preliminarily cooled down to -45°C so as to allow for uniform cooling, there could not be obtained a desired cooling curve having a supercooling point and a passing time through the maximum ice crystal formation zone of not less than five minutes presumably because of a rapid cooling rate. This required a long time before dissolution of the porous structure, and formation of lumps during the dissolution was found.

It will be noted that since the freezing conditions for obtaining a desired cooling curve differ depending on the amount of a polysaccharide aqueous solution and the type of cooling apparatus, they should not be limited to those of the above method of the examples and could be appropriately controlled by the expert in the art.

It is also to be noted that the present application is based on Japanese Patent Application No. 2009-203031, filed September 2, 2009, and the disclosure thereof is wholly incorporated herein by reference.

## Claims

**1.** A porous structure comprising a polysaccharide and having a multitude of pores wherein an average pore size of the pores is not smaller than 40 μm and a ratio of the number of pores having a pore size not smaller than 50 μm to the total number of the pores is not less than 30%.

**2.** A porous structure obtained by freeze-drying a polysaccharide aqueous solution containing a polysaccharide according to the following steps (A) and (B) :

the freezing step (A) of cooling said polysaccharide aqueous solution down to over a supercooling point and subsequently obtaining ice crystals of said polysaccharide aqueous solution via a time of passage through a maximum ice crystal formation zone of not less than five minutes; and
the drying step (B) of removing water contained in said ice crystals by sublimation under reduced pressure.

**3.** The porous structure as defined in Claim 1 or 2, wherein said polysaccharide has at least one selected from the group consisting of a substituted or unsubstituted carboxyl group, amino group and aldehyde group.

**4.** The porous structure as defined in any one of Claims 1 to 3, wherein a standard deviation σ of said pores is at 5 to 30 μm.

**5.** A method for preparing a porous structure by freeze-drying a polysaccharide aqueous solution according to the following steps (A) and (B):

the freezing step (A) of cooling said polysaccharide aqueous solution down to over a supercooling point and subsequently obtaining ice crystals of the polysaccharide aqueous solution via a time of passage through a maximum ice crystal formation zone of not less than five minutes; and
the drying step (B) of removing water contained in said ice crystals by sublimation under reduced pressure.

Fig. 1

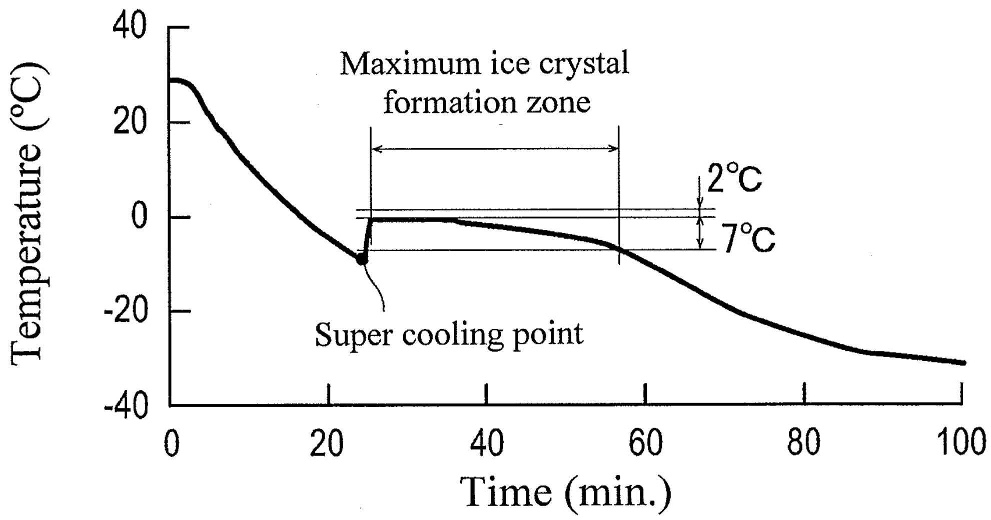
Maximum ice crystal formation zone
2°C
7°C
Super cooling point
Temperature (°C)
40
20
0
-20
-40
0
20
40
60
80
100
Time (min.)

Fig. 2

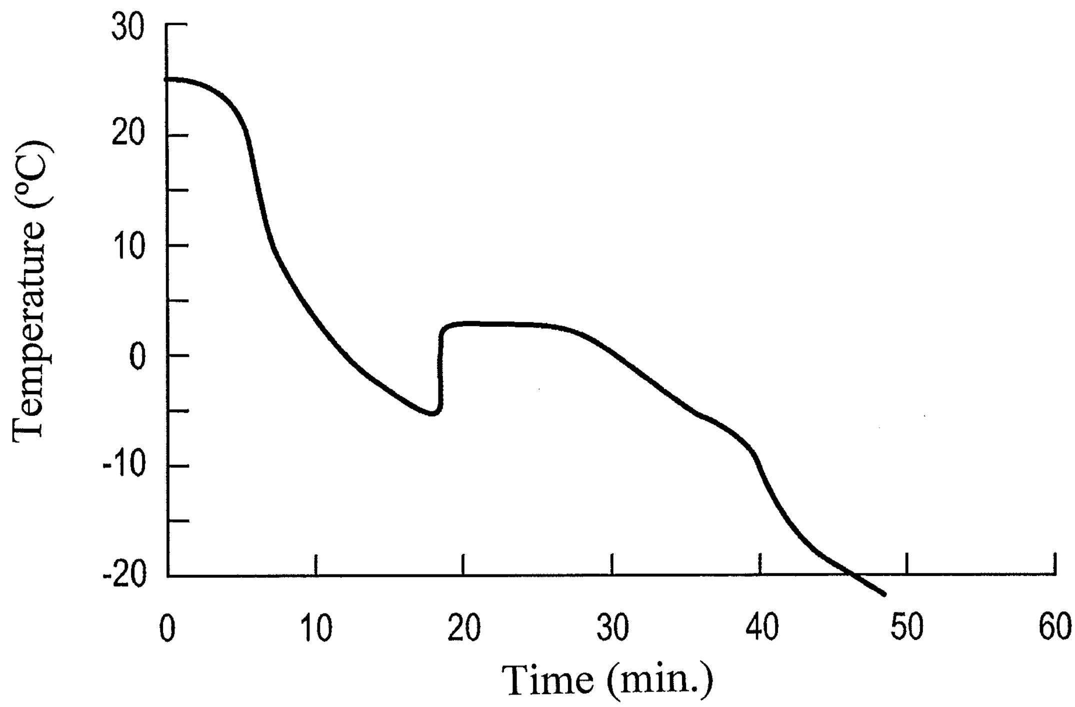
Temperature (°C)
30
20
10
0
-10
-20
0
10
20
30
40
50
60
Time (min.)

Fig. 3

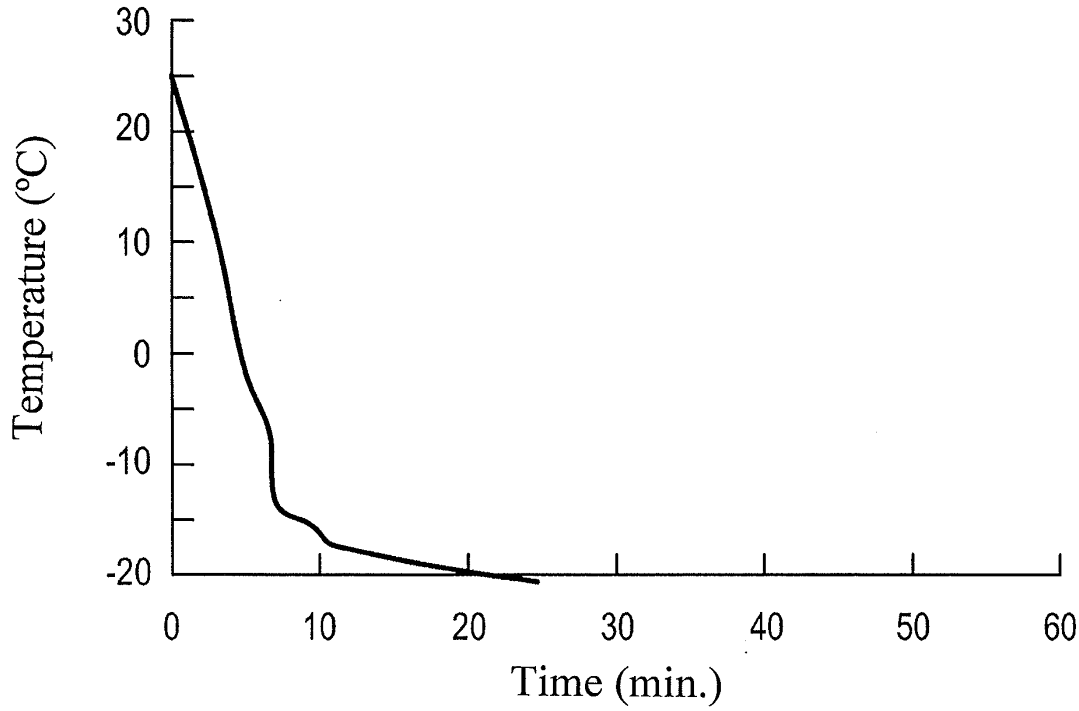
30
20
10
0
-10
-20
Temperature (ºC)
0
10
20
30
40
50
60
Time (min.)

Fig. 4

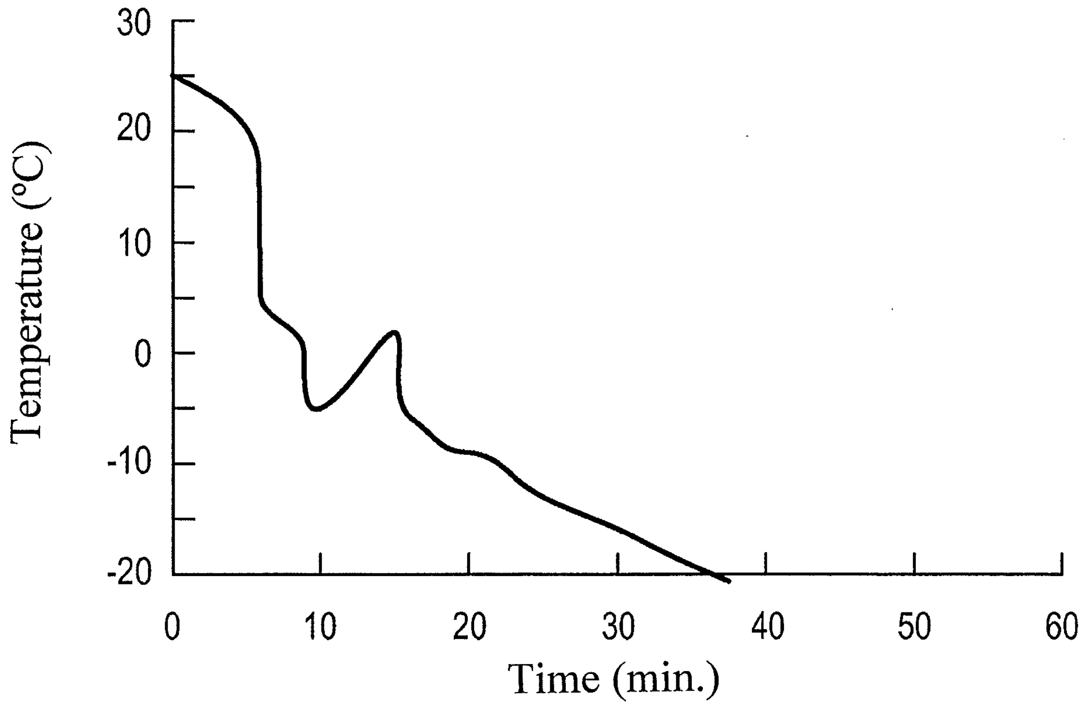
30
20
10
0
-10
-20
Temperature (ºC)
0
10
20
30
40
50
60
Time (min.)

Fig. 5

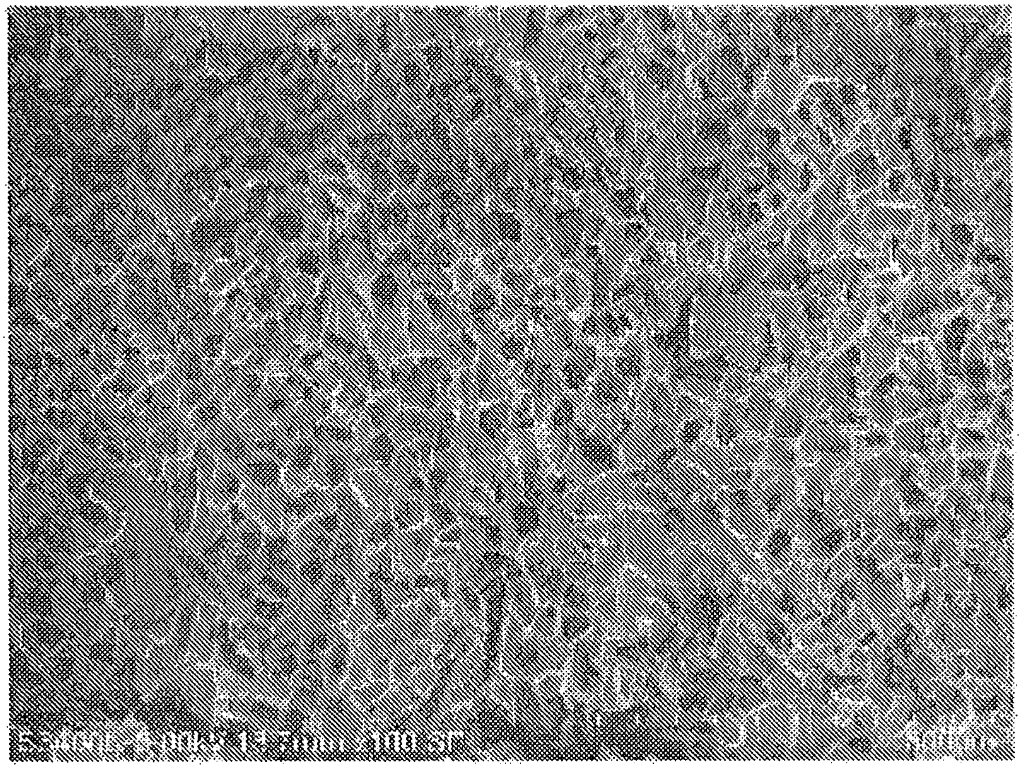

Fig. 6

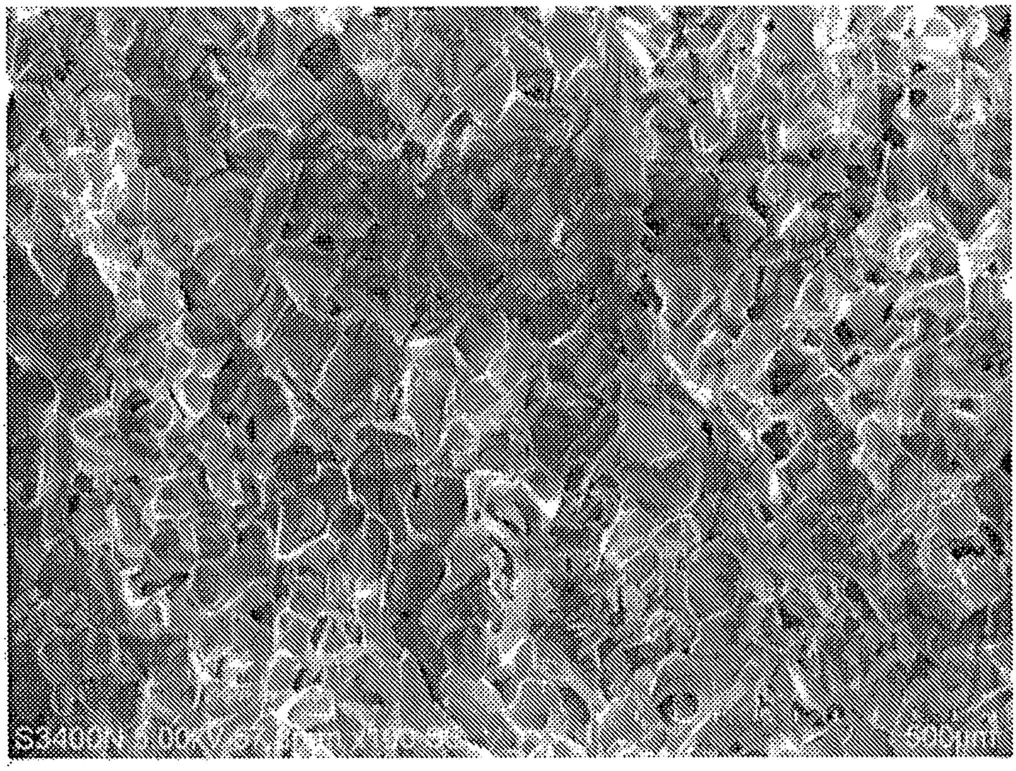

Fig. 7

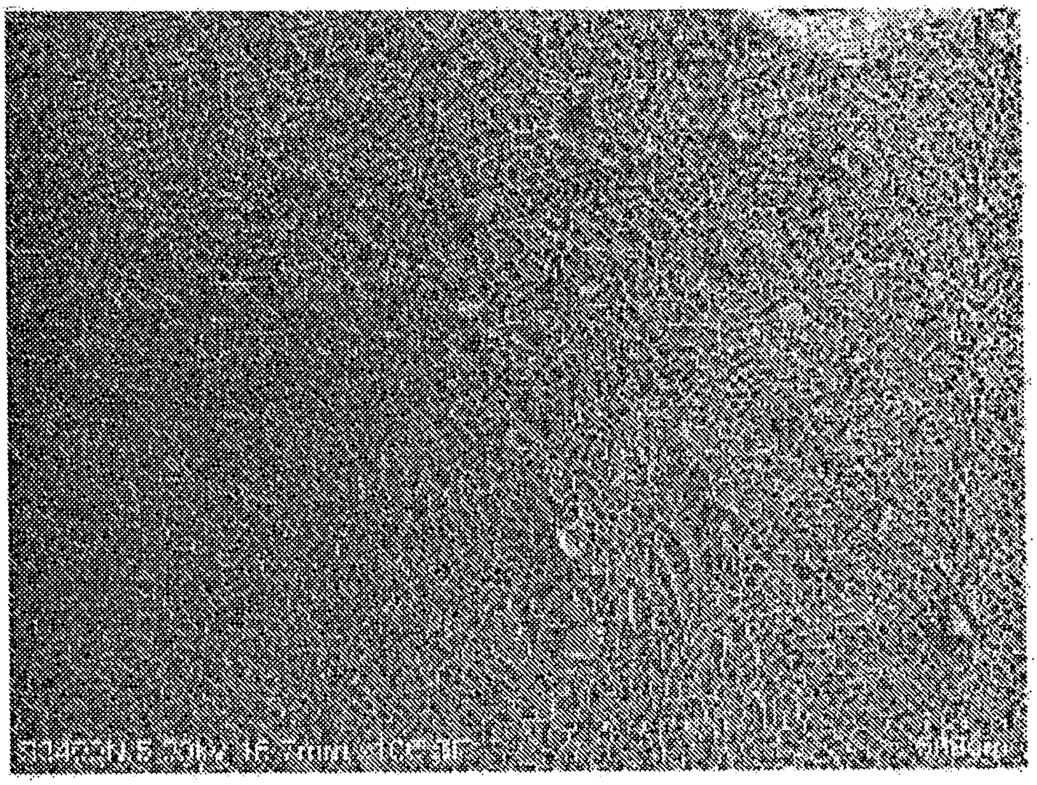

Fig. 8

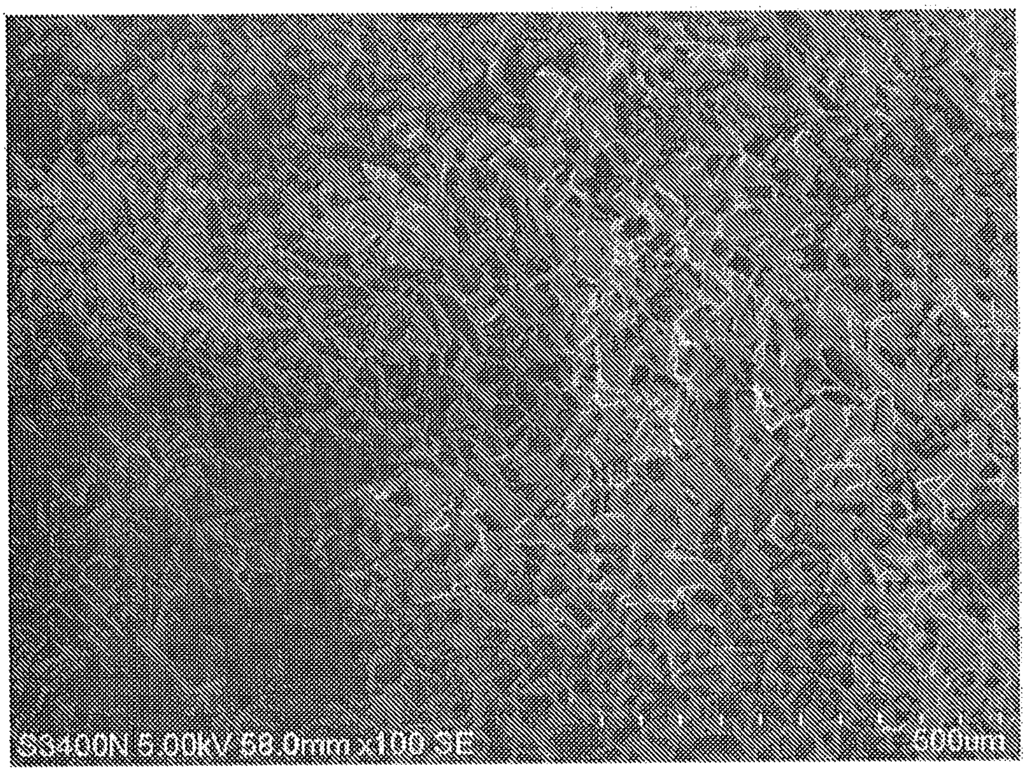

**INTERNATIONAL SEARCH REPORT**

International application No.
PCT/JP2010/064451

A. CLASSIFICATION OF SUBJECT MATTER
*C08J9/28*(2006.01)i, *A61K31/715*(2006.01)i, *A61P41/00*(2006.01)i

According to International Patent Classification (IPC) or to both national classification and IPC

B. FIELDS SEARCHED

Minimum documentation searched (classification system followed by classification symbols)
C08J9/00-42, A61K31/715, A61P41/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | WO 01/57121 A1 (Menicon Co., Ltd.),<br>09 August 2001 (09.08.2001),<br>claims; page 10, line 4 to page 18, line 22; examples; fig. 25<br>& US 2003/0015825 A1 & EP 1273615 A1 | 1,2,4,5<br>3 |
| Y<br>A | WO 2005/087289 A1 (Terumo Corp.),<br>22 September 2005 (22.09.2005),<br>claims; paragraphs [0036] to [0046], [0067] to [0074]; examples<br>& US 2008/0058469 A1 | 3<br>1,2,4,5 |
| A | JP 2003-1074 A (Daicel Chemical Industries, Ltd.),<br>07 January 2003 (07.01.2003),<br>entire text<br>(Family: none) | 1-5 |

☒ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 September, 2010 (07.09.10) | 21 September, 2010 (21.09.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No. PCT/JP2010/064451

C (Continuation). DOCUMENTS CONSIDERED TO BE RELEVANT

| Category* | Citation of document, with indication, where appropriate, of the relevant passages | Relevant to claim No. |
|---|---|---|
| A | JP 64-11141 A (Nitsupi Collagen Kogyo Kabushiki Kaisha), 13 January 1989 (13.01.1989), entire text (Family: none) | 1-5 |
| P,A | WO 2009/133763 A1 (Terumo Corp.), 05 November 2009 (05.11.2009), entire text (Family: none) | 1-5 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005087289 A
- US 2008058469 A
- JP 2008289986 A
- US 2008294099 A
- JP 2009203031 A